# EUROPÄISCHE PATENTSCHRIFT

(11) **EP 0 414 275 B1**
(45) Veröffentlichungstag und Bekanntmachung des Hinweises auf die Patenterteilung: **08.12.1993**
(21) Anmeldenummer: 90116293.3
(22) Anmeldetag: 24.08.1990
(51) Int. Cl.: C07C 229/28, C07C 227/22, C07C 227/10, C07C 255/46, C07C 253/30

(54) **Verfahren zur Herstellung von cyclischen Aminosäurederivaten sowie Zwischenprodukte**
Process for the preparation of cyclic aminoacid derivitives as well as the intermediate products
Procédé pour la préparation de dérivés d'aminoacides cycliques ainsi que les produits intermediaires

(30) Priorität: 25.08.1989 DE 3928184
(43) Veröffentlichungstag der Anmeldung: 27.02.1991
(73) Patentinhaber: GÖDECKE AKTIENGESELLSCHAFT, D-10587 Berlin (DE)
(72) Erfinder: Steiner, Klaus, Dr., D-7808 Waldkirch (DE); Herrmann, Wolfgang, Dr., D-7802 Merzhausen (DE); Crone, Günter, Dr., D-7800 Freiburg (DE); Combs, Charles Shepherd, Chester, New Jersey 07930 (US)

(56) Entgegenhaltungen:
- EP-A- 0 358 092
- CH-A- 444 146
- DE-A- 2 543 821
- JUSTUS LIEBIGS ANNALEN DER CHEMIE. vol. 688, 1965, WEINHEIM DE Seiten 113 - 121; HELMUT SCHÄFER: "EINE NEUE SYNTHESE ALPHA.ALPHA-DISUBSTITUIERTER SUCCINIMIDE"

## Beschreibung

Die vorliegende Erfindung betrifft ein Verfahren zur Herstellung von cyclischen Aminosäurederivaten und von deren pharmakologisch verträglichen Salzen der allgemeinen Formel I 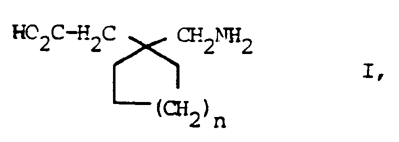
in welcher n eine ganze Zahl von 1 bis 3, bevorzugt 2, bedeutet.

Das Verfahren ist dadurch gekennzeichnet, das man ein Malonsäureesterderivat der allgemeinen Formel II 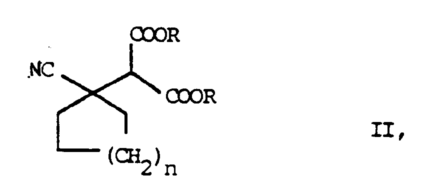
in welcher R eine niedere Alkylgruppe von 1 bis 5 Kohlenstoffatomen, bevorzugt eine Ethylgruppe, darstellt und n die obengenannte Bedeutung hat,
durch alkalische Hydrolyse in ein Cyanocycloalkylmalonsäurederivat der allgemeinen Formel III 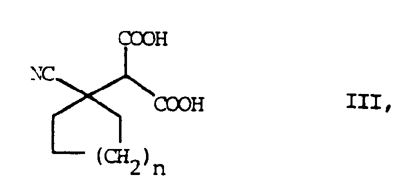
in welcher n die obengenannte Bedeutung hat,
überführt, dieses zu einem Zwischenprodukt der allgemeinen Formel IV 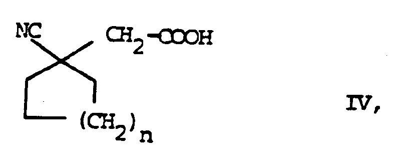
in welcher n die obengenannte Bedeutung hat,
decarboxyliert und die Nitrilgruppe in Gegenwart von Katalysatoren hydriert.

Die Verbindung in welcher n = 2 ist (Gabapentin, Drugs of the Future, Vol 9, Nr. 6, 1984, S. 418-419) ist die bisher am besten untersuchte Verbindung der allgemeinen Formel I. Sie wurde, ausgehend von Cyclohexanon bisher in einer umständlichen 7- bzw. 8-stufigen Synthese hergestellt.

Bei allen bisher bekannten Verfahren zur Herstellung von Gabapentin (vgl. DE-A 24 60 891) muß eine Synthesezwischenstufe durch saure Hydrolyse in wässrigem Medium in Gabapentin Hydrochlorid überführt werden. Dabei bildet sich aus Gabapentin durch Wasserabspaltung und intramolekulare Cyclisierung auch das Lactam (2-Azaspiro[4,5]decan-3-on) gemäß der allgemeinem Formel V 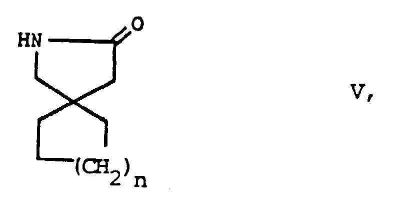
in welcher n die obengenannte Bedeutung hat,
als Nebenprodukt (vgl. EP-A 0 414 274).
Das anfallende Gabapentin-Hydrochlorid muß dann in verdünnter, wäßriger Lösung mittels Ionenaustauscher in Gabapentin überführt werden. Aus der so erhaltenen wässrigen Lösung kann das Gabapentin ohne nennenswerte Lactambildung nur durch technisch aufwendige Methoden erhalten werden. Die Umständlichkeit der Synthese, die unerwünschte Lactambildung sowie die aufwendige Isolierung des Gabapentins aus wässrigen Lösungen führen folglich zu einem hohen Herstellkostenpreis für Gabapentin.

Aufgabe der Erfindung ist es daher, ein ökonomisches und technisch durchführbares Verfahren zur Herstellung von Verbindungen der allgemeinen Formel I, insbesondere von Gabapentin zu entwickeln. Es sollte hierbei die Anzahl der Synthesestufen verringert und die unerwünschte Lactambildung unterbunden werden. Weiterhin sollte ein Verfahren entwickelt werden, das die Isolierung des Gabapentins aus nichtwässrigen Lösungen erlaubt.

Überraschenderweise wurde nun gefunden, daß diese Aufgabe durch die eingangs und in den Patentansprüchen beschriebenen Syntheseschritte gelöst werden kann. Nach nahezu quantitativer Überführung der Malonsäureesterderivate (II) durch alkalische Hydrolyse in die 1-Cyanocycloalkyl-malonsäurederivate (III) und durch schonende Decarboxylierung zur 1-Cyanocycloalkylessigsäure (IV), können diese in alkoholischen Lösungsmitteln mittels Katalysatoren direkt zu Verbindungen der Formel I hydriert werden. Durch die erfindungsgemäßen Reaktionsbedingungen kann hierbei die Bildung eines Lactams der allgemeinen Formel V verhindert werden. Durch das neue Verfahren entfällt das Überführen von Salzen der allgemeinen Formel I in die entsprechenden Basen sowie die aufwendige Isolierung der letzteren aus wässrigen Lösungen (Schema 1).

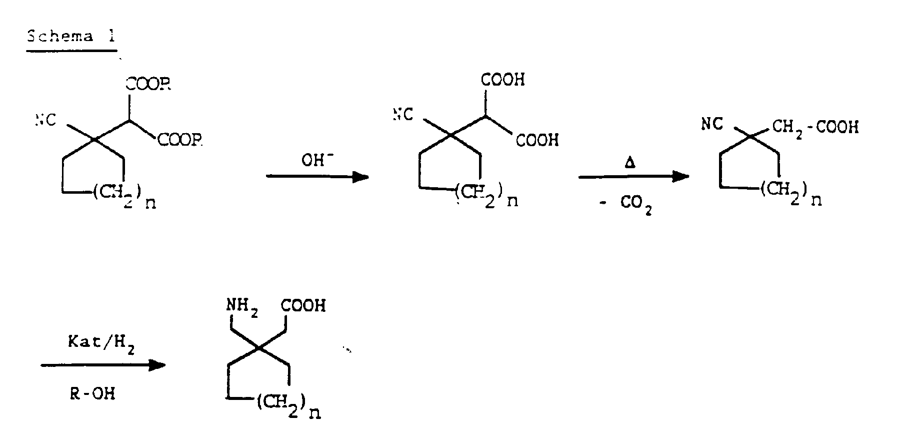

Als Katalysatoren können Raney-Nickel, Raney-Kobalt oder Edelmetallkatalysatoren wie Rhodium oder Palladium, eventuell auf einen Träger wie z.B. Kohle eingesetzt werden.

Die alkalische Hydrolyse der Verbindungen II und deren Überführung in ein Cyanocycloalkyl-malonsäurederivat der Formel III erfolgt üblicherweise entweder mittels Alkali- oder Erdalkalihydroxiden, bzw. mittels deren Salzen mit schwachen Säuren, wie z.B. Essigsäure oder Kohlensäure.

Als Hydrierungskatalysatoren kommen allgemein Raney-Nickel, Raney-Cobalt, Edelmetalle wie Platin, Palladium oder Rhodium ggf. auf üblichem Trägermaterial in Frage.

Die Decarboxylierung von Verbindungen der allgemeinen Formel III erfolgt entweder in der Schmelze oder in organischen Lösungsmitteln, wie z.B. in Ethylacetat, Toluol, Methylethylketon, Dioxan oder Hexan, niederen Alkoholen mit 1-8 Kohlenstoffatomen, sowie in halogenierten Kohlenwasserstoffen wie 3,3-Trichlorethylen. Die alkalische Hydrolyse der Verbindungen II wird bevorzugt in Alkoholen mit 1 bis 4 Kohlenstoffatomen bzw. in deren Mischungen mit Wasser durchgeführt.

Die Hydrierung der Verbindung IV wird bei einem Druck von 1-50 KPa und in einem relativ weiten Temperaturbereich zwischen Raumtemperatur und 80°C durchgeführt. Als bevorzugte niedere Alkohole werden Ethanol, Isopropanol und Butanol eingesetzt.

Die Erfindung wird durch die nachfolgenden Ausführungsbeispiele veranschaulicht und erläutert:

### Beispiel 1

(1-Cyanocyclohexyl)malonsäure

50 g (1-Cyanocyclohexyl)malonsäurediethylester werden in 175 ml Methanol bei 40°C gelöst. Bei dieser Temperatur wird eine Lösung von 29,9 g Natriumhydroxid in 150 ml Wasser zugetropft. Das Reaktionsgemisch wird 30 min bei 40°C gerührt. Nach Abkühlen auf 20°C wird das ausgefallene Produkt (Na-Salz) abgesaugt. Der Nutschkuchen wird mit 50 ml Methanol gewaschen. Das Produkt wird bei 60°C bis zur Gewichtskonstanz getrocknet. Das Produkt wird bei 10°C in 500 ml Wasser gelöst und unter Eiskühlung bei 10 - 15°C mit konz. Salzsäure auf pH 1 - 2 eingestellt. Die hierbei ausfallende Substanz wird 3 mal mit je 300 ml Essigester extrahiert. Die vereinigten Essigesterphasen werden getrocknet und am Rotationsverdampfer bei 35°C eingeengt. Der weiße Rückstand wird im Vakuumtrockenschrank bei 35°C bis zur Gewichtskonstanz getrocknet (35,5 g = 89,8 % d.Th; Fp.: 99,9°C).

### Beispiel 2

(1-Cyanocyclohexyl)essigsäure

35,5 g (1-Cyanocyclohexyl)malonsäure werden in 400 ml Toluol suspendiert und unter Rühren während ca. 1 h auf 80 - 85°C erhitzt. Im Verlauf der Decarboxylierung entsteht eine nahezu klare Lösung. Nach Filtration wird das Toluol bei 35°C im Vakuum abdestilliert. Das Rohprodukt wird in einer gesättigten Natriumbicarbonatlösung gelöst und mit Essigester verrührt. Nach Abtrennen der organischen Phase wird die wässrige Phase unter Eiskühlung bei 5°C mit konz. Salzsäure angesäuert. Das hierbei ausfallende Festprodukt wird abgesaugt und mit Wasser nachgewaschen. Der Nutschkuchen wird in Essigester gelöst und über Natriumsulfat getrocknet. Nach Filtration wird der Essigester am Rotationsverdampfer bei 35°C eingeengt. Der Rückstand wird im Vakuumtrockenschrank bei 35°C bis zur Gewichtskonstanz getrocknet (19,6g = 70 % d.Th., Fp.: 102°C).

### Beispiel 3

Gabapentin

Eine Lösung von 3 g (1-Cyanocyclohexyl)essigsäure in 10,5 ml Methanol wird bei 10 bar Wasserstoffdruck und 30°C über 0,2 g Rhodiumkohle (5 %ig) während 5 h hydriert. Das Reaktionsgemisch wird filtriert und am Rotationsverdampfer bei 30°C eingeengt. Der kristalline Rückstand wird mit Isopropanol verrührt. Das Kristallisat wird abgesaugt und im Vakuumtrockenschrank bei 30°C bis zur Gewichtskonstanz getrocknet (2,0 g = 65,2 % d.Th., Fp.: 152°C).

## Patentansprüche

1. Verfahren zur Herstellung von cyclischen Aminosäurederivaten und von deren pharmakologisch verträglichen Salzen der allgemeinen Formel I 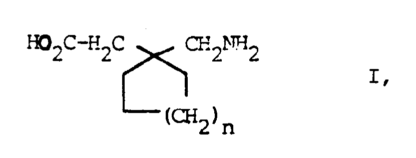 in welcher n eine ganze Zahl von 1 bis 3, bevorzugt 2, bedeutet,
dadurch gekennzeichnet, daß man ein Malonsäureesterderivat der allgemeinen Formel II 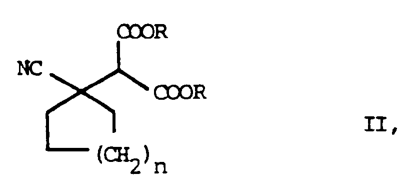 in welcher R eine niedere Alkylgruppe von 1 bis 5 Kohlenstoffatomen, bevorzugt eine Ethylgruppe, darstellt und n die obengenannte Bedeutung hat,
durch alkalische Hydrolyse in ein Cyanocycloalkylmalonsäurederivat der allgemeinen Formel III 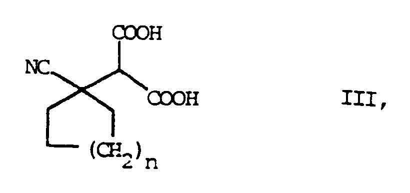 in welcher n die obengenannte Bedeutung hat,
überführt, dieses zu einem Zwischenprodukt der allgemeinen Formel IV 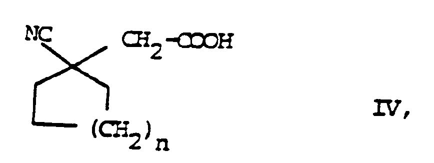 in welcher n die obengenannte Bedeutung hat,
decarboxyliert und die Nitrilgruppe in Gegenwart eines Katalysators hydriert.

2. Verfahren nach Anspruch 1, dadurch gekennzeichnet, daß man die Decarboxylierung in der Schmelze durchführt.

3. Verfahren nach Anspruch 1, dadurch gekennzeichnet, daß man die Decarboxylierung in inerten organischen Lösungsmitteln durchführt.

4. Verfahren nach Anspruch 1, dadurch gekennzeichnet, daß man die alkalische Hydrolyse in alkoholischer Lösung durchführt.

5. Verfahren nach Anspruch 1, dadurch gekennzeichnet, daß man die Hydrierung in alkoholischer Lösung, bei einem erhöhten Druck von bis zu 50 KPa und bei Raumperatur bis 80°C durchführt.

6. Verfahren nach Anspruch 1, dadurch gekennzeichnet, daß man die Hydrierung nach der Decarboxylierung und in einem getrennten Verfahrensschritt durchführt.

7. Verbindung der allgemeinen Formel IV gemäß Anspruch 1.

8. Verbindung gemäß Anspruch 7, wobei n in der allgemeinen Formel IV die Zahl 2 bedeutet.

9. Verfahren zur Herstellung einer Verbindung der allgemeinen Formel IV gemäß Anspruch 1, dadurch gekennzeichnet, daß man ein Malonsäureesterderivat der allgemeinen Formel II 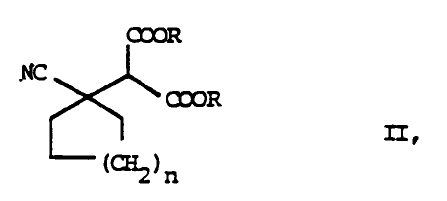 in welcher R eine niedere Alkylgruppe von 1 bis 5 Kohlenstoffatomen, bevorzugt eine Ethylgruppe, darstellt und n die obengenannte Bedeutung hat,
durch alkalische Hydrolyse in ein Cyanocycloalkylmalonsäurederivat der allgemeinen Formel III 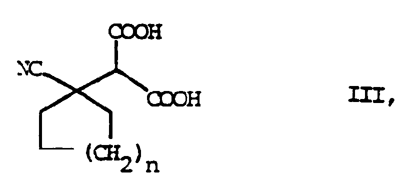 überführt und dieses decarboxyliert.

## Claims

1. Process for the preparation of cyclic amino acid derivatives and their pharmacologically compatible salts of the general formula I 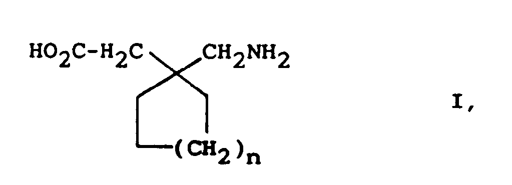 in which n signifies a whole number of 1 to 3, preferably 2, characterized in that a malonic acid ester derivative of the general formula II 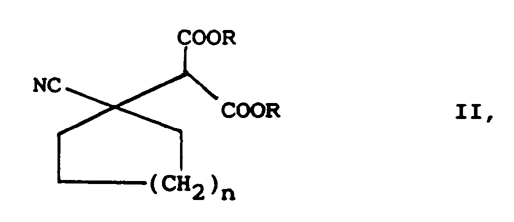 in which R represents a low alkyl group of 1 to 5 carbon atoms, preferably an ethyl group, and n has the aforementioned meaning, is converted by alkaline hydrolysis into a cyanocycloalkylmalonic acid derivative of the general formula III 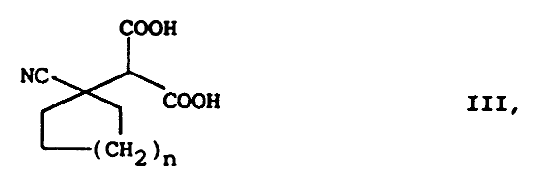 in which n has the aforementioned meaning, which is decarboxylated into an intermediate product of the general formula IV 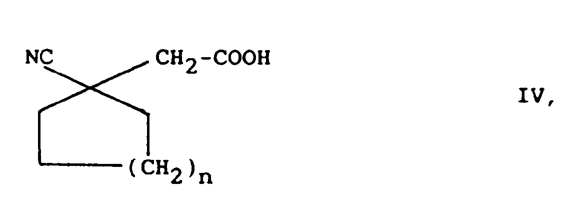 in which n has the aforementioned meaning, and the nitrile group is hydrogenated in the presence of a catalyst.

2. Process according to claim 1,
characterized in that the decarboxylation is carried out in the melt.

3. Process according to claim 1,
characterized in that the decarboxylation is carried out in inert organic solvents.

4. Process according to claim 1,
characterized in that the alkaline hydrolysis is carried out in alcoholic solution.

5. Process according to claim 1,
characterized in that the hydrogenation is carried out in alcoholic solution at an increased pressure of up to 50 KPa and at ambient temperature to 80°C.

6. Process according to claim 1,
characterized in that the hydrogenation is carried out after the decarboxylation and in a separate procedural step.

7. Compound of the general formula IV as defined in claim 1.

8. Compound according to claim 7, whereby n in the general formula IV signifies the number 2.

9. Process for the preparation of a compound of the general formula IV as defined in claim 1,
characterized in that a malonic acid ester derivative of the general formula II 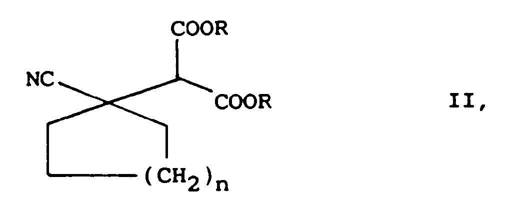 in which R represents a low alkyl group of 1 to 5 carbon atoms, preferably an ethyl group, and n has the aforementioned meaning, is converted by alkaline hydrolysis into a cyanocycloalkylmalonic acid derivative of the general formula III 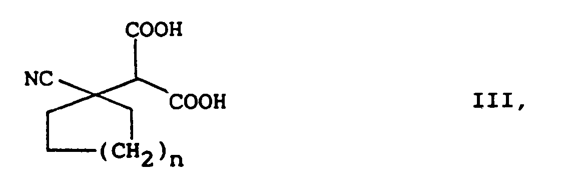 and this is decarboxylated.

## Revendications

1. Procédé de préparation de dérivés d'aminoacides cycliques et de leurs sels pharmacologiquement compatibles de formule générale I: 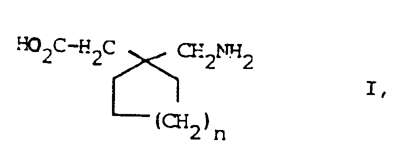 dans laquelle:
n représente un nombre entier de 1 à 3, de préférence 2,
ce procédé est caractérisé en ce que l'on fait passer un dérivé d'ester malonique de formule générale II: 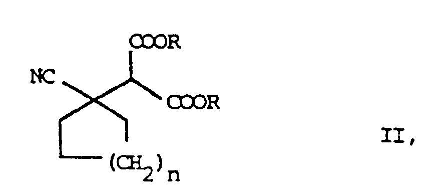 dans laquelle:
R représente un groupe alkyle inférieur comprenant de 1 à 5 atomes de carbone, de préférence un groupe éthyle, et
n a la signification indiquée ci-dessus,
par hydrolyse alcaline en un dérivé d'acide cyanocycloalkylmalonique de formule générale III: 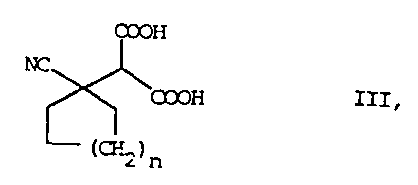 dans laquelle:
n présente la signification indiquée ci-dessus,
puis procède à la décarboxylation de celui-ci en un produit intermédiaire de formule générale IV: 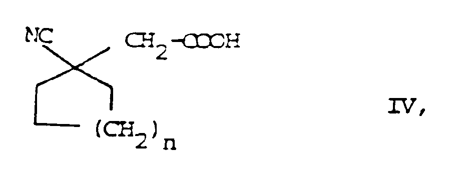 dans laquelle:
n présente la signification indiquée ci-dessus,
et à l'hydrogénation du groupe nitrile en présence d'un catalyseur.

2. Procédé selon la revendication 1, caractérisé en ce que la décarboxylation est mis en oeuvre à l'état fondu.

3. Procédé selon la revendication 1, caractérisé en ce que la décarboxylation est mis en oeuvre dans des solvants organiques inertes.

4. Procédé selon la revendication 1, caractérisé en ce que l'hydrolyse alcaline est mise en oeuvre dans une solution alcoolique.

5. Procédé selon la revendication 1, caractérisé en ce que l'hydrogénation est mise en oeuvre dans une solution alcoolique à une pression accrue atteignant jusqu'à 50 KPa et à une température comprise entre la température ambiante et 80°C.

6. Procédé selon la revendication 1, caractérisé en ce que l'hydrogénation est mise en oeuvre après la décarboxylation et dans une étape distincte.

7. Dérivé de formule générale IV selon la revendication 1.

8. Dérivé selon la revendication 7, caractérisé en ce que n, dans la formule générale IV, signifie le chiffre 2.

9. Procédé de préparation d'un dérivé de formule générale IV selon la revendication 1, caractérisé en ce que l'on transforme un dérivé d'ester d'acide malonique de formule générale II: 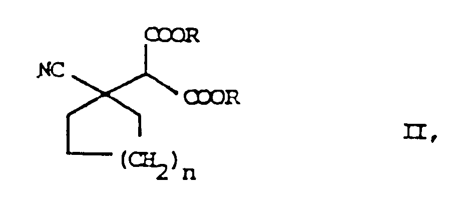 dans laquelle:
R représente un groupe alkyle inférieur renfermant de 1 à 5 atomes de carbone, de préférence un groupe éthyle, et
n présente la signification indiquée ci-dessus,
par hydrolyse alcaline en un dérivé d'acide cyanocycloalkylmalonique de formule générale III: 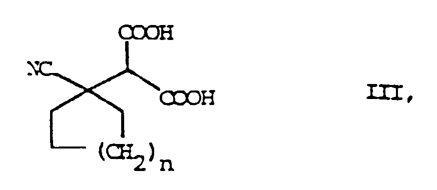 puis procède à la décarboxylation de celui-ci.
